# EUROPEAN PATENT APPLICATION

(11) **EP 0 933 428 A2**
(43) Date of publication of application: **04.08.1999**
(21) Application number: 97923309.5
(22) Date of filing: 06.05.1997
(51) Int. Cl.: C12N 15/54, C12N 15/82, C12N 9/10, C12N 9/16, A01H 5/00

(54) **METHOD FOR INCREASING THE CONTENT OF TREHALOSE IN ORGANISMS THROUGH THE TRANSFORMATION THEREOF WITH THE cDNA OF THE TREHALOSE-6-PHOSPHATE SYNTHETASE/PHOSPHATASE OF SELAGINELLA LEPIDOPHYLLA**

(30) Priority: 08.05.1996 MX 9601719
(71) Applicant: UNIVERSIDAD NACIONAL AUTONOMA DE MEXICO, Mexico 04510 D.F. (MX); K.U. Leuven Research & Development, 3000 Leuven (BE)
(72) Inventor: ITURRIAGA DE LA FUENTE, Gabriel, Cuernavaca, Morelos 62240 (MX); ZENTELLA GOMEZ, Rodolfo, Coyoacán, México, D.F. 14310 (MX)
(74) Representative: Van Someren, Petronella F. H. M.
(86) International application number: MX9700012
(87) International publication number: WO9742327

(57) **Abstract**

Abstract

## Description

Description

## Claims

1. Claims
